Europäisches Patentamt

European Patent Office

Office européen des brevets

(11) Veröffentlichungsnummer: **0 022 556**
A1

## (12) EUROPÄISCHE PATENTANMELDUNG

(21) Anmeldenummer: 80103931.4

(22) Anmeldetag: 09.07.80

(51) Int. Cl.³: **A 61 N 1/04,** H 01 B 1/02,
H 01 B 7/00, C 22 C 19/00,
C 22 C 38/00

(30) Priorität: 13.07.79 DE 2928394
03.09.79 DE 2935488

(43) Veröffentlichungstag der Anmeldung: 21.01.81
Patentblatt 81/3

(84) Benannte Vertragsstaaten: AT BE CH DE FR GB IT LI NL
SE

(71) Anmelder: Müller, Gerhard J., Prof. Dr.,
Bischof-Fischer-Strasse 106/108, D-7080 Aalen (DE)

(72) Erfinder: Müller, Gerhard J., Prof. Dr.,
Bischof-Fischer-Strasse 106/108, D-7080 Aalen (DE)

(74) Vertreter: Kraus, Walter, Dr., Patentanwälte Dres. Kraus
& Weisert Irmgardstrasse 15, D-8000 München 71 (DE)

(54) Implantierbarer elektrischer Leiter, insbesondere Stimulationselektrodenleitung und/oder -elektrode.

(57) Mit der Erfindung wird ein implantierbarer elektrischer Leiter zur Verfügung gestellt, und zwar eine amorphe Metallegierung, die sich insbesondere als Stimulationselektrodenleitung und/oder -elektrode eignet, weil sie eine hohe Biege-, Dauer-, Ermüdungs- und Wechselfestigkeit hat, womit lange Implantationszeiten von mehr als fünf und sogar mehr als zehn Jahren erreichbar sind, die auch durch die gute Korrosionsbeständigkeit bedingt sind. In einer besonderen Ausführungsform ist die amorphe Metallegierung weichmagnetisch und wird als Abschirmung einer implantierbaren elektronischen Schaltung verwendet.

EP 0 022 556 A1

# KRAUS & WEISERT

PATENTANWÄLTE

DR. WALTER KRAUS DIPLOMCHEMIKER · DR.-ING. ANNEKÄTE WEISERT DIPL.-ING. FACHRICHTUNG CHEMIE

IRMGARDSTRASSE 15 · D-8000 MÜNCHEN 71 · TELEFON 089/79 70 77-79 70 78 · TELEX 05-212156 kpat d

TELEGRAMM KRAUSPATENT

0022556

2617 JS/lh

PROF. DR. GERHARD MÜLLER

Aalen

---

## Implantierbarer elektrischer Leiter, insbesondere Stimulationselektrodenleitung und/oder -elektrode

---

Die Erfindung betrifft einen implantierbaren elektrischen Leiter, insbesondere eine Stimulationselektrodenleitung und/oder -elektrode.

Insbesondere bei der Implantation von Herzschrittmachern werden Stimulationselektrodenleitungen und -elektroden mit hoher Wechsellastfestigkeit und großer Korrosionsfestigkeit benötigt. Das menschliche Herz führt, wenn es mit einer Frequenz von 70 $min^{-1}$ von einem Herzschrittmacher angeregt wird, im Verlaufe eines Jahres 36,8 Millionen Kontraktionen aus. Zusätzlich zu der ventriculären Aktion erfolgt außerdem auch eine mehr oder weniger synchronisierte Bewegung des Atriums, und zwar zusammen mit einer Atembewegung, was zu einer sehr komplizierten Bewegung der Stimulationselektrode führt. Daher sind diese Elektroden aufgrund ihrer Biegungen sowie auch aufgrund von Torsion und Dehnung, welche letztere auch, wenngleich in einem zwar geringeren Maße, erfolgen, hohen dynamischen Belastungen ausgesetzt, wie im einzelnen von W. Greatbatch und W.M. Chardack in dem Aufsatz mit dem nachstehend in deutscher Übersetzung wiedergegebenen Titel "Myocardische und endocardische Elektroden für chronische Implantation" in Ann. N.Y. Acad. Sci. 148, 234-251 (1968) dargelegt ist. Diese außerordentlich hohen dynamischen Belastungen führten in der Vergangenheit oft zu Elektrodenbrüchen, insbesondere in der Nähe der Trikuspidalklappe. Es verging eine beträchtliche Zeit, bis einigermaßen brauchbare Elektroden entwickelt und ein bis zu einem gewissen Grad dauerhaftes Material verfügbar waren, mit denen eine sogenannte Langzeittherapie ermöglicht wurde. Die Zeitdauer dieser Langzeittherapie ist aber immer noch ziemlich beschränkt.

Die gleichen Schwierigkeiten treffen auch für die Stimulationselektrodenleitungen zu, da diese dauernden Wechselbelastungen ausgesetzt sind.

Bei myocardischen Stimulationselektrodenleitungen und -elektroden wurden zur Verbesserung der Wechsellastfestigkeit metallische Fäden, insbesondere Bänder, um einen flexiblen Kunststoffkern, beispielsweise aus Polyester, gewickelt. Bei endocardialen Stimulationselektrodenleitungen und -elektroden ergab sich eine gewisse Verbesserung der Flexibilität und Integrität durch Anwendung einer sogenannten "Spulenstruktur", bei der eine Hülse für eine Versteifungssonde vorhanden ist. Auf diese Weise wird eine transvenöse Anordnung beträchtlich erleichtert. Nachdem man eine geeignete Position gefunden hat, wird die Sonde aus der Hülse herausgezogen, und die Stimulationselektrodenleitung und -elektrode erhalten dadurch ihre volle Flexibilität.

Es wurden auch Versuche unternommen, die Stimulationselektrodenleitung und -elektrode zum Zwecke des Erzielen einer besseren Stabilität zu versteifen, indem man die Sonde in der Hülse ließ. Jedoch hat sich eine solche Maßnahme als gefährlich erwiesen, und zwar auch in den Fällen, in denen die Sonde aus rostfreiem Stahl besteht, weil sich herausgestellt hat, daß diese Sonde innerhalb von drei bis sechs Monaten aufgrund der Millionen von Funktionszyklen bricht. Die scharfen Bruchkanten, die beim Bruch einer solchen Sonde auftreten, reiben innerhalb der spiralförmig um die Sonde herum verlaufenden Stimulationselektrodenleitung und bewirken Ermüdungsbrüche derselben. Es hat sich beispielsweise herausgestellt, daß eine solche Stimulationselektrodenleitung nach etwa sechs Monaten brach, wobei die Sonde an einer Vielzahl von Stellen gebrochen war. Insbesondere kommt es dann zu Bruchstellen, wenn die Stimulationselektrodenleitung während der Implantation oder während eines Austauschs gequetscht wird, was sich nicht immer vermeiden läßt, auch wenn der die Implantation ausführende Chirurg sehr vorsichtig ist, insbesondere wenn er eine Klemme zum Halten der Stimulationselektrodenleitung bzw. -elektrode benutzt, und wenn er es weiterhin vermeidet, scharfe Schrauben zum Befestigen der Stimulationselektrodenleitung
an dem Herzschrittmacher zu verwenden.

Die vorerwähnten Schwierigkeiten konnten bis zu einem gewissen Grade dadurch vermindert werden, daß man eine Stimulationselektrodenleitung bzw. -elektrode entwickelte, die
nicht nur einen spiralförmig auf einen Kunststoffkern gewickelten Leiter aufwies, sondern vier solche Leiter, bei
denen die Bänder aus leitendem Material dünner als bei einem einzelnen Leiter und diese vier Leiter parallel geschaltet waren. In einem dieser Ausführungsbeispiele, das in dem
Buch, dessen Titel in deutscher Übersetzung "Fortschritte
in der Herzschrittmacher-Technologie" lautet und das von M.
Schaldach und S. Furman in Zusammenarbeit mit F. Hein und
R. Thull 1975 im Springer-Verlag Berlin Heidelberg New York
herausgegeben worden ist, auf Seite 242 beschrieben und abgebildet ist, sind diese vier Leiter parallel zueinander
sowie spiralförmig um einen Polyesterkern gewickelt. Auf
diese Weise konnten die Flexibilität und die Integrität
verbessert und der elektrische Widerstand herabgesetzt werden, und eine Funktion der Stimulationselektrode wurde erst
dann beeinträchtigt, wenn alle vier spiralförmigen Leitungen gebrochen waren. In diesem Zusammenhang sei auch auf
den Aufsatz von D.W. van Heeckeren, J.F. Hogan, W.W.L. Glenn
und M.S. Brooks hingewiesen, dessen Titel in deutscher Übersetzung "Technische Analyse von Herzschrittmacherelektroden"
lautet und der in Ann. N.Y. Acad. Sci. 167, 774-784 (1969)
erschienen ist.

Obwohl bei dieser zuletzt erwähnten redundanten Ausführungsform von Stimulationselektrodenleitungen eine gewisse Verbesserung erreicht werden konnte, haben diese redundanten
Stimulationselektrodenleitungen in der Praxis nicht zu den
erhofften Ergebnissen geführt, und zwar ist das vor allem
darauf zurückzuführen, daß die einzelnen spiralförmigen
Leitungen einer aus mehreren parallelen spiralförmigen Leitungen bestehenden Stimulationselektrodenleitung bzw.

-elektrode kein wirkliches redundantes System darstellen. Denn die wichtigste Bedingung, die von einem echten redundanten System zu erfüllen ist, besteht darin, daß die einzelnen redundanten Elemente vollständig unabhängig voneinander sein müssen, diese Bedingung ist bei dem erwähnten Aufbau nicht erfüllt. Wenn nämlich eine dieser spiralförmigen Leitungen bricht, werden die übrigen spiralförmigen Leitungen entsprechend höheren Beanspruchungen ausgesetzt; darüberhinaus vermindert sich der Widerstand der übrigen Leitungen gegen Knickung an der Stelle eines Bruchs einer dieser Leitungen beträchtlich. Außerdem sind die einzelnen Leitungen in ihrem Querschnitt gegenüber einer Leitung, die aus einem einzigen spiralförmigen Leiter besteht, erheblich vermindert. Obwohl bei einer aus mehreren spiralförmigen Leitern zusammengesetzten Leitung die gesamte Belastung an jeder Stelle auf die Mehrzahl von spiralförmigen Leitern verteilt wird, führt die normalerweise vorhandene verminderte Anzahl von Windungen jedes einzelnen spiralförmigen Leiters zu einer beträchtlichen Erhöhung der Torsionsbelastung, die umgekehrt zum Quadrat der Verminderung des Leiterradius zunimmt.

Es ist weiterhin versucht worden, die Schwierigkeiten, die sich einer Langzeitimplantation von Stimulationselektrodenleitungen und -elektroden entgegenstellen, durch Anwendung von speziellen Materialien zu lösen. Hierbei ist zu berücksichtigen, daß der Körper für alle Fremdmaterialien eine "Gastumgebung" darstellt, die sich den fremden Materialien abwehrend entgegenstellt. Infolgedessen entsteht im menschlichen Körper gegen alle implantierten Materialien eine Gewebegegenreaktion, die außerordentlich stark aber auch, je nach dem Material, so gering sein kann, daß sie als praktisch nicht vorhanden angesehen werden kann. Unter Berücksichtigung dieser Tatsachen wurden für Stimulationselektrodenleitungen und -elektroden bisher vor allem Platin, Platin-Iridium-Legierungen und eine unter dem Namen Elgiloy bekannte Legierung aus Kobalt, Chrom, Nickel, Molybdän und Eisen verwendet, und als Isolationsmaterialien zur Leitungsisolation Silicongummi, Polyäthylen und Polyurethan (siehe Seite 247 des oben erwähnten Buches "Fortschritte in der Herzschrittmacher-Technologie").

Trotzdem genügen die vorhandenen Stimulationselektrodenleitungen und -elektroden den heutigen Anforderungen nicht mehr, da nach der derzeitigen technischen Entwicklung Implantationszeiten von mehr als fünf Jahren, insbesondere von zehn Jahren und länger, zu fordern sind, weil es inzwischen Herzschrittmacher gibt, die als solche eine brauchbare Lebensdauer von zehn Jahren und mehr haben (siehe Seite 243 des oben erwähnten Buches "Fortschritte in der Herzschrittmacher-Technologie").

Mit der vorliegenden Erfindung soll daher ein implantierbarer elektrischer Leiter, insbesondere eine Stimulationselektrodenleitung und -elektrode, zur Verfügung gestellt werden, die den Anforderungen einer längen Implantationszeit entspricht, also insbesondere eine hohe Wechsellastfestigkeit und eine große Korrosionsfestigkeit besitzt; der elektrische Leiter soll in der Lage sein, möglichst selbst an Biegestellen und selbst an Stellen einer gewissen mechanischen Beschädigung, insbesondere Quetschung, die während der Implantation auftreten können, eine außerordentlich hohe Anzahl von wechselnden Belastungsbeanspruchungen auszuhalten, ohne zu brechen.

Es wurde überraschend gefunden, daß diese Bedingungen von einem metallischen Leiter erfüllt werden, der aus einer amorphen Metallegierung besteht, die auch als metallisches Glas oder Metallglas bezeichnet wird.

Eine solche korrosionsfeste, amorphe Metallegierung, die erfindungsgemäß als implantierbarer elektrischer Leiter, insbesondere Stimulationselektrodenleitung und/oder -elektrode, verwendet wird, hat neben hoher Festigkeit, insbesondere hoher Wechsellastfestigkeit und langer Lebensdauer sowie guter Verträglichkeit mit dem menschlichen Körpergewebe außerdem noch den Vorteil, daß sie aufgrund des speziellen Herstellungsverfahrens, das zur Erzielung des amorphen Zustands bei der Verfestigung der Metallegierung aus der Schmelze, angewandt wird, in dünnen und schmalen Bändern anfällt, die, wie sich aus den obigen Ausführungen ergibt, für die Herstellung von Stimulationselektrodenleitungen besonders geeignet sind.

Vorzugsweise wird als amorphe Metallegierung eine chromhaltiges Metallglas, insbesondere ein chromhaltiges Eisen-, Nickel- oder Stahlglas, verwendet, da diese Metallgläser außerordentlich korrosionsbeständig sind.

Das chromhaltige Metallglas kann insbesondere ein Metallglas der Formel $Fe_{32}Ni_{36}Cr_{14}P_{12}B_6$ sein, das eine sehr große Härte und eine hervorragende Korrosionsbeständigkeit besitzt und unter dem Warenzeichen "METGLAS" mit der Legierungsbezeichnung 2826A von der Firma Allied Chemical vertrieben wird; diese Metallglaslegierung ist nichtmagnetisch, und ihre Korrosionsbeständigkeit ist so groß, daß sie beispielsweise von einer 6%igen $FeCl_3$-Lösung bei $60^oC$ während zwanzig Stunden nicht angegriffen wird.

Ein weiteres chromhaltiges Metallglas großer Korrosionsbeständigkeit ist amorphes $FeCrP_{13}C_7$ bzw. $FeCr_xP_{13}C_7$, dessen Korrosionsgeschwindigkeit in 1N Natriumchlorid bei $30^oC$ bei einem Chromgehalt von 2,5% bei 0,10 mm pro Jahr, bei einem Chromgehalt von 4% bei 0,05 mm pro Jahr und bei einem Chromgehalt von 8 und mehr Prozent praktisch bei 0 mm pro Jahr liegt.

Der bandförmige, implantierbare elektrische Leiter gemäß der Erfindung kann mit Kunststoff isoliert werden, und zwar insbesondere mit Silicongummi bzw. -kautschuk, Polyäthylen oder Polyurethan, wobei dieser bandförmige Leiter im einfachsten Fall mit einem dazu konzentrischen Kunststoffmantel elektrisch isoliert ausgeführt sein kann.

Zu bevorzugen ist es jedoch, daß der als Band vorliegende elektrische Leiter spiralförmig gewickelt und mit dem Kunststoff isoliert ist. Das kann in verschiedener Weise geschehen, beispielsweise so, daß der Kunststoff einen Kunststoff-Hohlkern, insbesondere einen Kunststoffschlauch, bildet, innerhalb von dessen Mantel das Band sowohl nach der Außenseite des Kunststoff-Hohlkerns als auch nach dessen Innerem hin elektrisch isoliert ist. Diese Ausführungsform ermöglicht es, durch den Kunststoff-Hohlkern ein Mandrel, eine Sonde o.dgl. zu führen, mit dem die Stimulationselektrodenleitung und -elektrode in geeigneter Weise im Körper positioniert werden kann.

Während die vorerwähnte Ausführungsform, bei der das Band in den Mantel eines Kunststoff-Hohlkörpers eingebettet ist, einen gewissen herstellungsmäßigen Aufwand erfordert, weil das spiralförmig gewickelte Band in diesem Falle freitragend mit Kunststoff umspritzt werden muß, kann es bei der Herstellung kleinerer Mengen von implantierbaren Stimulationselektrodenleitungen nach der Erfindung vorteilhaft sein, die Herstellung dadurch zu vereinfachen, daß das Band um einen Kunststoffkern gewickelt und dann elektrisch isoliert wird, wobei der Kunststoffkern sowohl ein Kunststoff-Vollkern als auch ein Kunststoff-Hohlkern, insbesondere ein Kunststoffschlauch sein kann. Das um den Kunststoffkern gewickelte Band kann dann anschließend mit einem zum Kern konzentrischen Kunststoffmantel, insbesondere mit einem Kunststoffschlauch, nach außen hin elektrisch isoliert werden.

Wenn man eine Doppelwegleitung benötigt, läßt sich die vorerwähnte Ausführungsform nach der Erfindung dahingehend weiterbilden, daß man um ein erstes mit Kunststoff isoliertes Band, das als Kern dient, ein zweites Band wickelt und es gegebenenfalls beispielsweise in der zuletzt erwähnten
Weise nach außen hin elektrisch isoliert, wobei das erste
mit Kunststoff isolierte Band ein auf einen Kunststoff-
Hohlkern, insbesondere einen Kunststoffschlauch, aufgewickeltes Band sein kann, in dessen hohles Innere ein
Mandrel, eine Sonde o.dgl. zu dem oben erwähnten Zweck
eingeführt werden kann.

Außer den Stimulationselektrodenleitungen und -elektroden
besitzen Herzschrittmacher eine hochempfindliche elektronische Schaltung, welche die Taktimpulse für die Stimulation des Herzens erzeugt, die über die erwähnten Stimulationselektrodenleitungen und -elektroden zum Herzen des
Trägers eines solchen Herzschrittmachers übertragen werden.
Derartige implantierbare, hochempfindliche elektronische
Schaltungen, von denen die elektronische Schaltung eines
Herzschrittmachers hier nur als bevorzugtes Beispiel angegeben ist, ändern ihre Kenndaten, wenn sie in magnetische
Felder kommen, wie sie zum Beispiel in der Nähe von elektrischen Großmaschinen oder Transformatoren auftreten.  Im Beispielsfall einer hochempfindlichen elektronischen Schaltung
eines Herzschrittmachers kann infolgedessen, wenn der Träger des Herzschrittmachers in ein stärkeres magnetisches
Feld der beispielsweise erwähnten Art kommt, die Funktion
dieser elektronischen Schaltung so stark beeinflußt oder
sogar unterbrochen werden, daß das letale Folgen für den
Träger des Herzschrittmachers hat.

Bisher ist es nicht gelungen, eine ausreichende magnetische
Abschirmung für elektronische Schaltungen von Herzschrittmachern oder für andere implantierbare elektronische Schaltungen zu finden.  Deshalb ist es im Beispielsfalle von Herzschrittmacherträgern notwendig, daß sich diese von den Quellen starker magnetischer Felder fernhalten, da diese Felder für die Träger von Herzschrittmachern, wie erwähnt, lebensgefährlich sind. Trotz der strikten Anweisung, die Träger von Herzschrittmachern haben, starke magnetische Felder zu meiden, kann es doch vorkommen, daß der Träger eines Herzschrittmachers zufälligerweise in ein starkes magnetisches Feld gerät. Außerdem ist es für eine ganze Reihe von Herzschrittmacherträgern aus beruflichen Gründen erwünscht, daß die elektronische Schaltung ihres Herzschrittmachers gegen stärkere Magnetfelder abgeschirmt ist.

Es wurde nun überraschenderweise gefunden, daß sich die Gefährdung eines Trägers einer implantierbaren elektronischen Schaltung durch ein Magnetfeld dadurch beseitigen läßt, daß ein implantierbarer elektrischer Leiter aus einer weichmagnetischen, amorphen Metallegierung verwendet und als magnetische Abschirmung der implantierbaren elektronischen Schaltung ausgebildet wird.

Derartige weichmagnetische, amorphe Metallegierungen haben eine hohe Abschirmungswirkung, denn ihre maximale Permeabilität liegt sehr hoch, und ihre Abschirmwirkung wird durch mechanische Einwirkungen, insbesondere Erschütterungen, Stöße o.dgl., nicht verschlechtert.

Da solche weichmagnetischen, amorphen Metallegierungen aufgrund des speziellen Herstellungsverfahrens, das zur Erzielung des amorphen Zustands bei der Verfestigung der Metallegierung aus der Schmelze angewandt wird, in dünnen und schmalen Bändern anfallen, ist es zu bevorzugen, die magnetische Abschirmung der implantierbaren elektronischen Schaltung dadurch auszubilden, daß die aus dünnen, schmalen Bändern bestehende weichmagnetische, amorphe Metallegierung um die implantierbare elektronische Schaltung herumgewickelt wird oder daß sie in Form eines Geflechts um die implantierbare elektronische Schaltung herum vorgesehen ist, also letztere gewissermaßen in einem solchen Geflecht "verpackt" ist.

Die weichmagnetische, amorphe Metallegierung kann vorzugsweise eine Eisen-Legierung, insbesondere $Fe_{82}B_{18}$, eine Eisen-Nickel-Legierung, insbesondere $Fe_{40}Ni_{40}B_{20}$ oder $Fe_{40}Ni_{40}P_{14}B_6$, eine Cobalt-Nickel-Eisen-Legierung, insbesondere $Co_{50}Ni_{20}Fe_6Si_{12}B_{12}$, und/oder eine Cobalt-Eisen-Legierung, insbesondere $Co_{74}Fe_6B_{20}$, sein.

Bei der vorliegenden erfindungsgemäßen Anwendung von weichmagnetischen, amorphen Metallegierungen, die auch als metallische Gläser oder Metallgläser bezeichnet werden, braucht nicht notwendigerweise die Forderung nach Korrosionsbeständigkeit gestellt zu werden, da die von diesen weichmagnetischen, amorphen Metallegierungen bestehende Abschirmung gegebenenfalls auch innerhalb des herkömmlichen, korrosionsfesten Gehäuses der implantierbaren elektronischen Schaltung verwendet werden kann.

Ende der Beschreibung.

Patentansprüche

1.     Implantierbarer elektrischer Leiter, insbesondere
Stimulationselektrodenleitung und/oder -elektrode, dadurch
g e k e n n z e i c h n e t , daß der metallische Leiter
aus einer amorphen Metallegierung besteht.

2.     Implantierbarer elektrischer Leiter nach Anspruch 1,
dadurch g e k e n n z e i c h n e t , daß die amorphe Metall-
legierung ein chromhaltiges Metallglas, insbesondere ein
chromhaltiges Eisen-, Nickel- oder Stahlglas, vorzugsweise
ein Metallglas der Formel $Fe_{32}Ni_{36}Cr_{14}P_{12}B_6$ oder ein Metallglas der Formel $FeCr_xP_{13}C_7$ ist.

3.     Implantierbarer elektrischer Leiter nach Anspruch 1
oder 2, dadurch g e k e n n z e i c h n e t , daß die amorphe
Metallegierung die Form eines dünnen, schmalen Bandes hat, das
vorzugsweise eine Breite von 1 mm oder weniger sowie eine
Dicke von 0,1 mm oder weniger besitzt.

4.     Implantierbarer elektrischer Leiter nach Anspruch 3,
dadurch g e k e n n z e i c h n e t , daß das Band mit Kunststoff, insbesondere mit einem dazu konzentrischen Kunststoffmantel, elektrisch isoliert ist.

5.     Implantierbarer elektrischer Leiter nach Anspruch 4,
dadurch g e k e n n z e i c h n e t , daß das Band spiralförmig gewickelt und mit dem Kunststoff so isoliert ist, daß
dieser einen Kunststoff-Hohlkern, insbesondere einen Kunststoffschlauch bildet, innerhalb von dessen Mantel das Band
sowohl nach der Außenseite des Kunststoff-Hohlkerns als auch
nach dessen Innerem hin elektrisch isoliert ist.

6.      Implantierbarer elektrischer Leiter nach Anspruch 4, dadurch g e k e n n z e i c h n e t , daß das Band um einen Kunststoffkern gewickelt und elektrisch isoliert ist, wobei der Kunststoffkern ein Kunststoff-Vollkern oder ein Kunststoff-Hohlkern, insbesondere ein Kunststoffschlauch, ist.

7.      Implantierbarer elektrischer Leiter nach Anspruch 4, 5 oder 6, dadurch g e k e n n z e i c h n e t , daß um ein erstes mit Kunststoff isoliertes Band als Kern ein zweites Band gewickelt und gegebenenfalls elektrisch isoliert ist, oder daß das um den Kern gewickelte Band durch einen zum Kern konzentrischen Kunststoffmantel, insbesondere Kunststoffschlauch, elektrisch isoliert ist, wobei vorzugsweise bei Verwendung eines Kunststoff-Hohlkerns durch diesen ein Mandrel, eine Sonde o.dgl. geführt ist.

8.      Implantierbarer elektrischer Leiter nach einem der Ansprüche 1 bis 7, dadurch g e k e n n z e i c h n e t, daß er eine Stimulationselektrodenleitung eines Herzschrittmachers ist und/oder eine Elektrode eines Herzschrittmachers ist oder aufweist.

9.      Implantierbarer elektrischer Leiter nach Anspruch 1, dadurch g e k e n n z e i c h n e t , daß der implantierbare elektrische Leiter aus einer weichmagnetischen, amorphen Metallegierung besteht und als magnetische Abschirmung einer implantierbaren elektronischen Schaltung ausgebildet ist, wobei vorzugsweise die weichmagnetische, amorphe Metallegierung um die implantierbare elektronische Schaltung herumgewickelt oder in Form eines Geflechts um die implantierbare elektronische Schaltung herum vorgesehen ist.

10.     Implantierbarer elektrischer Leiter nach Anspruch 9, dadurch g e k e n n z e i c h n e t , daß die weichmagnetische, amorphe Metallegierung eine Eisenlegierung, insbesondere $Fe_{82}B_{18}$ oder eine Eisen-Nickel-Legierung, insbesondere

$Fe_{40}Ni_{40}B_{20}$ oder $Fe_{40}Ni_{40}P_{14}B_6$ oder eine Cobalt-Nickel-
Eisen-Legierung, insbesondere $Co_{50}Ni_{20}Fe_6Si_{12}B_{12}$, oder eine
Cobalt-Eisen-Legierung, insbesondere $Co_{74}Fe_6B_{20}$ ist.

Europäisches
Patentamt

**EUROPÄISCHER RECHERCHENBERICHT**

0022556

Nummer der Anmeldung

EP 80103931.4

| | **EINSCHLÄGIGE DOKUMENTE** | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - A1 - 2 708 151 (ALLIED CHEMICAL) <br><br> + Patentansprüche 1,5-7 + <br><br> -- | 1,9, 10 | A 61 N 1/04 <br> H 01 B 1/02 <br> H 01 B 7/00 <br> C 22 C 19/00 <br> C 22 C 38/00 |
| | DE - A1 - 2 613 072 (SIEMENS) <br><br> + Patentansprüche 1-5; Seite 3, Zeile 36 bis Seite 4, Zeile 10 + <br><br> -- | 1,8 | |
| | DE - A1 - 2 553 003 (ALLIED CHEMICAL) <br><br> + Seite 2, Zeile 4 - Seite 3, Zeile 25; Seite 5, Zeile 12 - Seite 6, Zeile 6; Seite 7, Zeilen 4-10 + <br><br> -- | 1-3, 9,10 | **RECHERCHIERTE SACHGEBIETE (Int. Cl.³)** <br><br> A 61 B <br> A 61 N <br> C 22 C <br> H 01 B |
| | DE - A - 2 319 500 (MEDTRONIC) <br><br> + Patentansprüche 1-4,6-9,16; Fig. 1 + <br><br> -- | 1,4-8 | |
| | FR - A1 - 2 257 700 (THE RESEARCH INSTITUT) <br><br> + Seite 2, Zeilen 5-7; Seite 3, Zeilen 3-14; Seite 14, Zeilen 21-34 + <br><br> -- | 1,2,9, 10 | |
| | FR - A1 - 2 376 218 (ALLIED CHEMICAL) <br><br> + Seite 5, Zeile 1 - Seite 8, Zeile 19; Seite 11, Zeile 22 - Seite 12, Zeile 20; Seite 14, Zeile 22 - Seite 15, Zeile 11; Seite 16, Zeilen 12-19; Patentansprüche 1-9 + <br><br> -- <br><br> ·/· | 1-3,9, 10 | **KATEGORIE DER GENANNTEN DOKUMENTE** <br><br> X: von besonderer Bedeutung <br> A: technologischer Hintergrund <br> O: nichtschriftliche Offenbarung <br> P: Zwischenliteratur <br> T: der Erfindung zugrunde liegende Theorien oder Grundsätze <br> E: kollidierende Anmeldung <br> D: in der Anmeldung angeführtes Dokument <br> L: aus andern Gründen angeführtes Dokument |
| X | Der vorliegende Recherchenbericht wurde für alle Patentansprüche erstellt. | | &: Mitglied der gleichen Patent- familie, übereinstimmendes Dokument |

| Recherchenort | Abschlußdatum der Recherche | Prüfer |
|---|---|---|
| WIEN | 07-11-1980 | NEGWER |

EPA form 1503.1  06.78

| | EINSCHLÄGIGE DOKUMENTE | | KLASSIFIKATION DER ANMELDUNG (Int. Cl.³) |
|---|---|---|---|
| K.t gorie | Kennzeichnung des Dokuments mit Angabe, soweit erforderlich, der Maßgeblichen Teile | betrifft Anspruch | |
| | US - A - 4 150 981 (ALLIED CHEMICAL) <br><br> + Spalte 5, Zeilen 18-32; Tabelle II + <br><br> -- | 1,9,10 | |
| | PATENT ABSTRACTS OF JAPAN, un-examined applications, Sektion C, Band 3, Nr. 68 (C-48), 13. Juni 1979, <br><br> The Patent Office Japanese Government, Seite 35 C 48 <br><br> + Kokai-Nr. 54-51221 (HITACHI) + <br><br> ---- | 1,9,10 | **RECHERCHIERTE SACHGEBIETE (Int. Cl.³)** |